# EUROPEAN PATENT APPLICATION

(11) **EP 4 215 270 A1**
(43) Date of publication of application: **26.07.2023**
(21) Application number: 21872717.0
(22) Date of filing: 12.08.2021
(51) Int. Cl.: B01J 23/89, B01J 23/63, B01J 23/46, B01J 23/42, B01J 37/02, C07D 307/46

(54) **CATALYST FOR PREPARING DICARBOXYLIC ACID AROMATIC HETEROCYCLIC COMPOUND, AND MEHTOD FOR PREPARING DICARBOXYLIC ACID AROMATIC HETEROCYCLIC COMPOUND**

(30) Priority: 22.09.2020 KR 20200122489; 06.08.2021 KR 20210103999
(71) Applicant: Kolon Industries, Inc., Seoul 07793 (KR)
(72) Inventor: JEONG, Mee Hye, Seoul 07793 (KR); KIM, Jun Yeong, Seoul 07793 (KR); YONG, Da Kyoung, Seoul 07793 (KR); HA, Ji Min, Seoul 07793 (KR); PARK, Ki Hyun, Seoul 07793 (KR)
(74) Representative: Ter Meer Steinmeister & Partner
(86) International application number: PCT/KR2021/010730
(87) International publication number: WO 2022/065687

(57) **Abstract**

Provided is a heterogeneous catalyst for preparing a dicarboxylic acid aromatic heterocyclic compound, the heterogeneous catalyst including an active metal; and a basic metal oxide carrier capable of carrying the active metal and forming a complex by coordinate bonding with the dicarboxylic acid aromatic heterocyclic compound.

## Description

### [Technical Field]

The present invention relates to a catalyst for preparing a dicarboxylic acid aromatic heterocyclic compound and a method for preparing a dicarboxylic acid aromatic heterocyclic compound.

### [Background Art]

In order to reduce greenhouse gases, developed countries such as the United States and Europe are strengthening carbon dioxide emission regulations. Therefore, there is an increasing demand for the biochemical industry that can reduce dependence on existing fossil raw material resources and reduce greenhouse gases. In other words, the chemical industry is changing from petroleum-dependent to bio-dependent.

For example, a technology for replacing polyethylene terephthalate (PET) mainly used as a beverage bottle or a food storage container with bio-PET is being actively researched. The bio-PET is prepared by usefully using a dicarboxylic acid aromatic heterocyclic compound as a monomer, which is a derivative usually prepared by oxidizing 5-hydroxymethyl furfural (HMF). The 5-hydroxymethyl furfural is obtained from sugar and thus a derivative of raw materials widely available in nature.

For reference, the 5-HMF may have an oxidation reaction, as shown in Reaction Scheme 1, obtaining an aromatic heterocyclic compound of monocarbonic acid or dicarboxylic acid including 2,5-furandicarboxylic acid (FDCA) and 5-formyl-2-furoic acid (FFCA) as a main product.

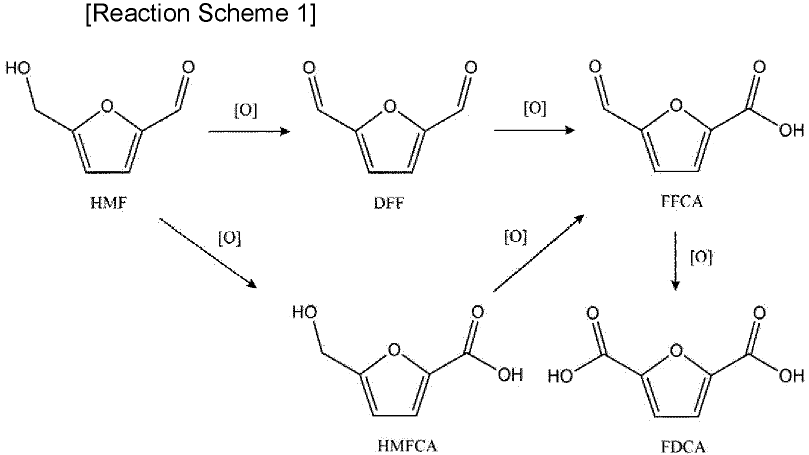

The oxidation of HMF in which the aromatic heterocyclic compound of monocarbonic acid or dicarboxylic acid is obtained as a main product is a process known in the literature. For example, U.S. Patent No. 4,977,283 (Hoechst) describes a method of oxidizing HMF by a metal catalyst belonging to a platinum group under an aqueous environment.

In addition, U.S. Patent Publication No. 2008-0103318 (Battelle) describes another method of oxidizing HMF catalyzed by platinum supported on a carrier.

However, these HMF oxidation methods need a high preparation cost and are carried out as a batch reaction, wherein the batch reaction has problems of requiring time substantially unrelated to the reaction such as input of raw materials, heating to a reaction temperature, discharge of reaction products, and the like and thus deteriorating productivity of a reactor. In addition, there are problems of requiring a process of separating a catalyst from the products in order to repeatedly use the catalyst and prematurely deteriorating activity of the catalyst due to changes in a temperature and the like.

### [Prior Art Documents]

### [Patent Document]

US Patent No. 4,977,283 (December 11, 1990)
US Patent Publication No. 2008-0103318 (May 1, 2008)

### [Disclosure]

### [Description of the Drawings]

### [Technical Problem]

An embodiment provides a catalyst for preparing a dicarboxylic acid aromatic heterocyclic compound in a reaction of preparing FDCA by oxidizing HMF, which may maintain a final product of FDCA in a liquid phase until the reaction is completed when a continuous process is applied thereto, improve a reaction rate to increase process efficiency, reduce side reactions of the catalyst such as contribution to production of H₂O₂ and conversion into CO or CO₂ to improve a yield, and be reused to dissolve FDCA in water in the purification process.

Another embodiment provides a method for preparing a dicarboxylic acid aromatic heterocyclic compound.

### [Technical Solution]

According to an embodiment, a heterogeneous catalyst for preparing a dicarboxylic acid aromatic heterocyclic compound includes an active metal and a basic metal oxide carrier capable of carrying the active metal and forming a complex by coordinate bonding with the dicarboxylic acid aromatic heterocyclic compound.

The active metal may include Fe, Ni, Ru, Rh, Pd, Os, Ir, Au, Pt, or a combination thereof.

The heterogeneous catalyst may include 0.1 wt% to 10 wt% of the active metal based on the total weight of the heterogeneous catalyst.

The heterogeneous catalyst may further include a catalyst enhancer including Bi, Na, Cu, Ce, K, or a combination thereof.

The heterogeneous catalyst may include a shell including the catalyst enhancer on the surface of the active metal.

The basic metal oxide capable of forming a complex by coordinate bonding with the dicarboxylic acid aromatic heterocyclic compound may include BaO, Nd₂O₃, Cs₂O, CsX (X is OH, Cl, Br, or I), basic zeolite, basic composite metal oxide, metal-organic frameworks (MOFs), hydrotalcite, hydroxyapatite, or a combination thereof.

The basic zeolite may be a zeolite in which hydrogen of a high silica beta or mordenite acidic zeolite having a silica/alumina (SiO₂/Al₂O₃) molar ratio of 20 to 1000 is substituted with an ion including Na, K, Rb, Cs, Mg, Ca, Sr, Ba, or a combination thereof.

The basic composite metal oxides may include Mg₂Al₂O₄, ZnAl₂O₄, BaTiO₃, ZnTiO₃, MgO-SiO₂, CaO-SiO₂, SrO-SiO₂, BaO-SiO₂, MgO-Al₂O₃, CaO-Al₂O₃, SrO-Al₂O₃, BaO-Al₂O₃, MgO-TiO₂, CaO-TiO₂, SrO-TiO₂, BaO-TiO₂, MgO-ZnO, CaO-ZnO, SrO-ZnO, BaO-ZnO, or a combination thereof.

The metal-organic frameworks may include Zr-based MOFs, Mg-based MOFs, Ca-based MOFs, Sr-based MOFs, Ba-based MOFs, or a combination thereof.

The hydrotalcite may include [Mg₄Al₂(OH)₁₂(CO₃)·4H₂O], [Mg₆Al₂(OH)₁₆(CO₃)·4H₂O], [Mg₄Zn₂Al₂(OH)₁₆(CO₃)·4H₂O], [Ca₄Al₂(OH)₁₂(NO₃)₂·xH₂O], [Ca₆Al₂(OH)₁₆(NO₃)₂·xH₂O], [Ca₄Zn₂Al₂(OH)₁₆(CO₃)·4H₂O], or a combination thereof.

The heterogeneous catalyst may produce a dicarboxylic acid aromatic heterocyclic compound represented by Chemical Formula 6 by oxidizing an aromatic heterocyclic compound represented by Chemical Formula 2 in a polar solvent.

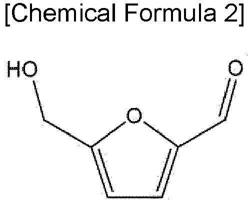

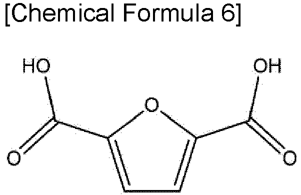

According to another embodiment, a method of preparing a dicarboxylic acid aromatic heterocyclic compound includes preparing a dicarboxylic acid aromatic heterocyclic compound by oxidizing a raw material for an aromatic heterocyclic compound in a polar solvent in the presence of a heterogeneous catalyst, wherein the heterogeneous catalyst includes an active metal and a basic metal oxide carrier capable of carrying the active metal and forming a complex by coordinate bonding with the dicarboxylic acid aromatic heterocyclic compound.

The aromatic heterocyclic compound raw material may be represented by Chemical Formula 1.

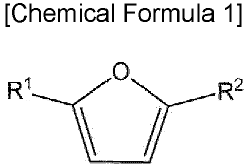

In Chemical Formula 1, one of R¹ and R² is an aldehyde group, and the other is a hydrogen group, a hydroxyl group, an aldehyde group, an acetoxy group, a halogen group, a substituted or unsubstituted C₁-C₂₀ alkyl group, or a substituted or unsubstituted C₁-C₂₀ alkoxy group.

The aromatic heterocyclic compound raw material represented by Chemical Formula 1 may include a compound represented by Chemical Formula 2, a compound represented by Chemical Formula 3, a compound represented by Chemical Formula 4, a compound represented by Chemical Formula 5, or a combination thereof.

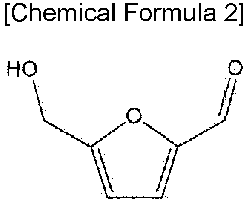

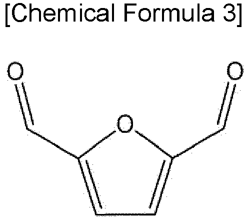

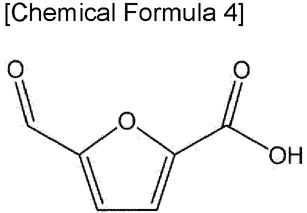

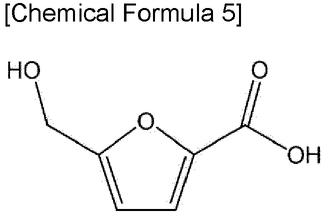

The dicarboxylic acid aromatic heterocyclic compound may be 2,5-furandicarboxylic acid (FDCA) represented by Chemical Formula 6.

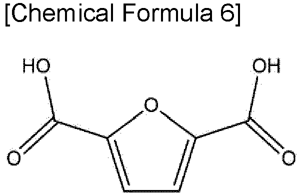

### [Advantageous Effects]

The catalyst for preparing a dicarboxylic acid aromatic heterocyclic compound according to an embodiment in a reaction of preparing FDCA by oxidizing HMF may maintain a final product of FDCA in a liquid phase until the reaction is completed when a continuous process is applied thereto, improve a reaction rate to increase process efficiency, reduce side reactions of the catalyst such as contribution to producing H₂O₂, conversion into CO or CO₂, or the like to improve a yield, and be reused for dissolving FDCA in water in the purification process.

### [Mode for Invention]

The advantages and features of the present disclosure and the methods for accomplishing the same will be apparent from the embodiments described hereinafter. However, the embodiments should not be construed as being limited to the embodiments set forth herein. Unless otherwise defined, all terms (including technical and scientific terms) used herein have the same meaning as commonly understood by one of ordinary skill in the art. In addition, terms defined in a commonly used dictionary are not interpreted ideally or excessively unless specifically and explicitly defined. Throughout the specification, when a part "includes" a certain component, it means that it may further include other components without excluding other components unless otherwise specified. Unless specifically described to the contrary, a singular form includes a plural form.

In the present specification, unless otherwise specified, the term "alkyl" refers to a saturated monovalent aliphatic hydrocarbon radical, including linear and branched chains, having the specified number of carbon atoms. The alkyl group typically contains from 1 to 20 carbon atoms ("C₁-C₂₀ alkyl"), specifically from 1 to 12 carbon atoms ("C₁-C₁₂ alkyl"), and more specifically from 1 to 8 carbon atoms ("C₁-C₈ alkyl"), or from 1 to 6 carbon atoms ("C₁-C₆ alkyl"), or 1 to 4 carbon atoms ("C₁-C₄ alkyl"). Examples of the alkyl group include methyl, ethyl, n-propyl, isopropyl, n-butyl, iso-butyl, tert-butyl, n-pentyl, isopentyl, neopentyl, n-hexyl, n-heptyl, n-octyl, and the like. The alkyl group may be substituted or unsubstituted. In particular, unless otherwise specified, the alkyl group may be substituted with one or more halogens, up to the total number of hydrogen atoms present in the alkyl moiety. Thus, C₁-C₄ alkyl includes halogenated alkyl groups, for example fluorinated alkyl groups having 1 to 4 carbon atoms, such as trifluoromethyl (-CF₃) or difluoroethyl (-CH₂CHF₂).

In the present specification, unless otherwise specified, the term "alkoxy" refers to a monovalent -O-alkyl group in which the alkyl moiety has the specified number of carbon atoms. The alkoxy group typically includes 1 to 8 carbon atoms ("C₁-C₈ alkoxy"), or 1 to 6 carbon atoms ("C₁-C₆ alkoxy"), or 1 to 4 carbon atoms ("C₁-C₄ alkoxy"). For example, C₁-C₄ alkoxy includes methoxy (-OCH₃), ethoxy (-OCH₂CH₃), isopropoxy (-OCH(CH₃)₂), tert-butyloxy (-OC(CH₃)₃), and the like. The alkoxy group is unsubstituted or substituted on the alkyl moiety by the same groups described herein as suitable for alkyl. In particular, an alkoxy group may be optionally substituted with one or more halo atoms, in particular one or more fluoro atoms, up to the total number of hydrogen atoms present in the alkyl moiety. Such groups are referred to as "haloalkoxy" having the specified number of carbon atoms and substituted with one or more halo substituents, for example when fluorinated, more specifically "fluoroalkoxy" groups, typically such groups contain from 1 to 6 carbon atoms, specifically 1 to 4 carbon atoms, often 1 or 2 carbon atoms, and 1, 2 or 3 halo atoms (i.e., "C₁-C₆ haloalkoxy," "C₁-C₄ haloalkoxy" or "C₁-C₂ haloalkoxy"). More specifically, a fluorinated alkyl group may be specifically referred to as a fluoroalkoxy group, typically substituted with 1, 2 or 3 fluoro atoms, such as a C₁-C₆, C₁-C₄, or C₁-C₂ fluoroalkoxy group. Thus, C₁-C₄ fluoroalkoxy includes trifluoromethyloxy (-OCF₃), difluoromethyloxy (-OCF₂H), fluoromethyloxy (-OCFH₂), difluoroethyloxy (-OCH₂CF₂H), and the like.

In the present specification, the term "aromatic" or "aryl," unless otherwise specified, refers to an optionally substituted monocyclic or fused bicyclic or polycyclic ring system having the well-known character of aromaticity, wherein one or more rings contain fully conjugated pi-electron systems. Typically, an aryl group has 6 to 20 carbon atoms ("C₆-C₂₀ aryl"), specifically 6 to 14 carbon atoms ("C₆-C₁₄ aryl"), more specifically 6 to 12 carbon atoms ("C₆-C₁₂ aryl"). A fused aryl group can include an aryl ring (e.g., a phenyl ring) fused to another aryl or heteroaryl ring or fused to a saturated or partially unsaturated carbocyclic or heterocyclic ring, provided that the points of attachment to the base molecules on these fused ring systems are atoms of the aromatic moiety of the ring system. For example, aryl groups include phenyl, biphenyl, naphthyl, anthracenyl, phenanthrenyl, indanyl, indenyl, or tetrahydronaphthyl. The aryl group is unsubstituted or substituted as further described herein.

As used herein, unless otherwise specified, the term "aromatic heterocycle" or "heteroaryl" refers to monocyclic or fused bicyclic or polycyclic ring systems with the well-known character of aromaticity which includes the specified number of ring atoms as a ring member in an aromatic ring and includes at least one heteroatom selected from N, O, and S. The inclusion of heteroatoms allows for aromaticity in the 5- and 6-membered rings. Typically, a heteroaryl group has 5 to 20 ring atoms ("5- to 20-membered heteroaryl"), specifically 5 to 14 ring atoms ("5- to 14-membered heteroaryl"), or more specifically 5- to 12-membered ring atoms ("5- to 12-membered heteroaryl"). The heteroaryl ring is attached to the base molecule through a ring atom of the heteroaromatic ring such that the aromaticity is maintained. Thus, a 6-membered heteroaryl ring may be attached to the base molecule through a ring C atom, whereas a 5-membered heteroaryl ring may be attached to the base molecule through a ring C or N atom. The heteroaryl group may also be fused to other aryl or heteroaryl rings, or fused to saturated or partially unsaturated carbocyclic or heterocyclic rings, wherein the point of attachment to the base molecule on such a fused ring system is an atom of the heteroaromatic moiety of the ring system. For example, the unsubstituted heteroaryl group may include pyrrole, furan, thiophene, pyrazole, imidazole, isoxazole, oxazole, isothiazole, thiazole, triazole, oxadiazole, thiadiazole, tetrazole. pyridine, pyridazine, pyrimidine, pyridine, benzofuran, benzothiophene, indole, benzimidazole, indazole, quinoline, isoquinoline, purine, triazine, naphthyridine, or carbazole. In various embodiments, the 5- or 6-membered heteroaryl group is selected from pyrrolyl, furanyl, thiophenyl, pyrazolyl, imidazolyl, isoxazolyl, oxazolyl, isothiazolyl, thiazolyl, triazolyl, pyridinyl, pyrimidinyl, pyrazinyl, or pyridazinyl rings. The heteroaryl groups are substituted or unsubstituted as further described herein.

As used herein, the term "halo" or "halogen" refers to fluorine (F), chlorine (Cl), bromine (Br), or iodine (I) unless otherwise specified.

In the present specification, the terms "optionally substituted" and "substituted or unsubstituted" are used interchangeably to indicate that a particular group being described may have no non-hydrogen substituents (i.e., unsubstituted), or the group may have one or more non-hydrogen substituents (i.e., substituted). Unless otherwise specified, the total number of substituents that may be present may equal the number of H atoms present on the unsubstituted form of the described group. When an optional substituent is attached through a double bond (e.g., an oxo (=O) substituent), the group occupies the available valency and the total number of other substituents included is reduced by two. When optional substituents are independently selected from a list of alternatives, the selected groups are the same or different. Throughout this specification, it will be understood that the number and nature of optional substituents will be limited to such extent that such substitutions satisfy chemical sense.

Suitable optional substituents for the alkyl, alkoxy, aryl, heteroaryl, and heterocyclyl ring may include, but are not limited to, C₁-C₈ alkyl, C₂-C₈ alkenyl, C₂-C₈ alkynyl, C₃-C₈ cycloalkyl, 3- to 12-membered heterocyclyl, C₆-C₁₂ aryl, and 5- to 12-membered heteroaryl, halo, =O (oxo), =S (thiono), =N-CN, =N-OR^{x}, =NR^{x}, -CN, -C(O)R^{x}, -CO₂R^{x}, -C(O)NR^{x}R^{y}, -SR^{x}, -SOR^{x}, -SO₂R^{x}, -SO₂NR^{x}R^{y}, - NO₂, -NR^{x}R^{y}, -NR^{x}C(O)R^{y}, -NR^{x}C(O)NR^{x}R^{y}, -NR^{x}C(O)OR^{x}, -NR^{x}SO₂R^{y}, - NR^{x}SO₂NR^{x}R^{y}, -OR^{x}, -OC(O)R^{x}, and -OC(O)NR^{x}R^{y}, wherein R^{x} and R^{y} are each independently hydrogen (H), C₁-C₈ alkyl, C₁-C₈ acyl, C₂-C₈ alkenyl, C₂-C₈ alkynyl, C₃-C₈ cycloalkyl, 3- to 12-membered heterocyclyl, C₆-C₁₂ aryl, 5- to 12-membered heteroaryl, or R^{x} and R^{y} together with the N atom to which they are attached may form a 3- to 12-membered heterocyclyl or 5- to 12-membered heteroaryl ring, each may optionally include one, two, or three additional heteroatoms selected from O, N, and S(O)_{q} (wherein q is 0 to 2); each of R^{x} and R^{y} is optionally substituted with one to three substituents selected from halo, =O, =S, =N-CN, =N-OR', =NR', -CN, -C(O)R', -CO₂R', -C(O)NR'₂, -SOR', -SO₂R', - SO₂NR'₂, -NO₂, -NR'₂, -NR'C(O)R', -NR'C(O)NR'₂, -NR'C(O)OR', -NR'SO₂R', - NR'SO₂NR'₂, -OR', -OC(O)R', and -OC(O)NR'₂, wherein each R' is each independently hydrogen (H), C₁-C₈ alkyl, C₁-C₈ acyl, C₂-C₈ alkenyl, C₂-C₈ alkynyl, C₃-C₈ cycloalkyl, 3- to 12-membered heterocyclyl, C₆-C₁₂ aryl, or C₅-C₁₂ heteroaryl; and each of the C₁-C₈ alkyl, C₂-C₈ alkenyl, C₂-C₈ alkynyl, C₃-C₈ cycloalkyl, 3- to 12-membered heterocyclyl, C₆-C₁₂ aryl, or C₅-C₁₂ heteroaryl may be optionally substituted as additionally defined.

In the present specification, "metal precursor" is a concept that refers to a chemical substance prepared in advance to react a metal. For example, a "transition metal precursor" is a concept that refers to a chemical substance prepared in advance to react a transition metal.

A heterogeneous catalyst for preparing a dicarboxylic acid aromatic heterocyclic compound according to an embodiment includes an active metal and a basic metal oxide carrier carrying the active metal.

The heterogeneous catalyst produces a dicarboxylic acid aromatic heterocyclic compound by an oxidation reaction of an aromatic heterocyclic compound raw material represented by Chemical Formula 1.

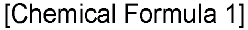

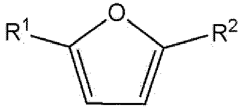

The aromatic heterocyclic compound raw material represented by Chemical Formula 1 is a compound converted from wood, and is a compound that is first used in the production of dicarboxylic acid aromatic heterocyclic compounds. For example, the aromatic heterocycle may be one or more selected from furan, thiophene, and pyrrole.

In Chemical Formula 1, one of R¹ and R² may be an aldehyde group, and the other may be a hydrogen group, a hydroxyl group, an aldehyde group, an acetoxy group, a halogen group, a substituted or unsubstituted C₁-C₂₀ alkyl group, or a substituted or unsubstituted C₁-C₂₀ alkoxy group. That is, in Chemical Formula 1, when R¹ is an aldehyde and R² is a hydrogen group, a hydroxyl group, an aldehyde group, an acetoxy group, a halogen group, a substituted or unsubstituted C₁-C₂₀ alkyl group, or a substituted or unsubstituted C₁-C₂₀ alkoxy group. Also, for example, the C₁-C₂₀ alkyl group or C₁-C₂₀ alkoxy group may be unsubstituted or substituted with one or more halogen atoms.

Specifically, the aromatic heterocyclic compound raw material may include compounds represented by Chemical Formula 2 to Chemical Formula 5, or a combination thereof.

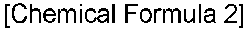

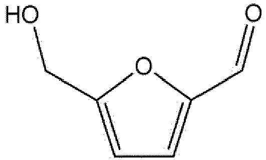

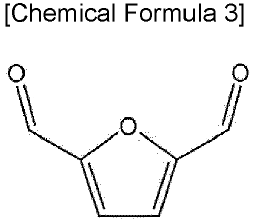

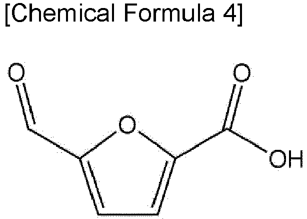

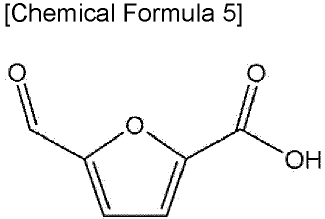

More specifically, the heterogeneous catalyst may oxidize 5-hydroxymethyl furfural (HMF) represented by Chemical Formula 2 and produce 2,5-furandicarboxylic acid (FDCA) represented by Chemical Formula 6, for example, through the same route as in Reaction Scheme 1.

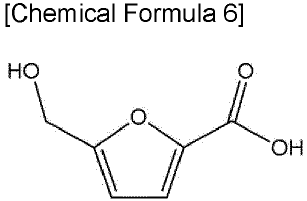

To this end, the heterogeneous catalyst includes an active metal and a basic metal oxide carrier carrying the active metal.

The active metal is a component having activity contributing to improving a production yield of the dicarboxylic acid aromatic heterocyclic compound from the aromatic heterocyclic compound raw material and supported on the basic metal oxide carrier.

The active metal may include Fe, Ni, Ru, Rh, Pd, Os, Ir, Au, Pt, or a combination thereof, which may have various oxidation numbers, and for example, include Ru, Pd, Pt, or Au.

The heterogeneous catalyst may include 0.1 wt% to 10 wt%, for example 0.1 wt% to 5 wt% of the active metal based on the total weight of the heterogeneous catalyst. When the content of the active metal is less than 0.1 wt%, the oxidation reaction may not proceed, but when the content of the active metal is greater than 10 wt%, which exceeds an amount required for the continuous process reaction, this unnecessary amount may bring about an economical loss, and the active metal may be not uniformly supported (deposited) on the carrier but agglomerated with one another.

The heterogeneous catalyst may further include a catalyst enhancer including Bi, Na, Cu, Ce, K, or a combination thereof along with the active metal. The catalyst enhancer may be supported with the active metal on the basic metal oxide carrier. In addition, the heterogeneous catalyst may include a shell including the catalyst enhancer on the active metal surface. The shell including the catalyst enhancer may be positioned on the basic metal oxide carrier.

The catalyst enhancer may promote separation of carbon monoxide (CO) or carbon dioxide (CO₂) on the heterogeneous catalyst surface, preventing activity deterioration of the heterogeneous catalyst. In addition, the catalyst enhancer may have a limited effect on a particle size of the active metal but greatly increase an amount of adsorbed hydrogen, nitrogen, and ammonia, and particularly, K may not only improve adsorption characteristics of the hydrogen, nitrogen, ammonia, and the like to Ru supported on a carrier.

The heterogeneous catalyst may include 0.1 wt% to 6 wt% of the catalyst enhancer based on the total weight of the heterogeneous catalyst. When the content of the catalyst enhancer is less than 0.1 wt%, the effect of preventing the activity deterioration of the heterogeneous catalyst by promoting the separation of carbon monoxide (CO) or carbon dioxide CO₂ on the heterogeneous catalyst surface may be insignificant, but when the content of the catalyst enhancer is greater than 6 wt%, the content of the active metal is reduced, deteriorating catalyst activity.

The basic metal oxide carrier includes a basic metal oxide capable of forming a complex by coordinate bonding with the dicarboxylic acid aromatic heterocyclic compound.

For example, the dicarboxylic acid aromatic compound may be prepared by oxidizing the aromatic heterocyclic compound raw material under the heterogeneous catalyst in a polar solvent, wherein the produced dicarboxylic acid aromatic heterocyclic compound is white powder but should exist in a liquid phase during the reaction to be not precipitated in a reactor. Herein, the basic metal oxide coordinates with the dicarboxylic acid aromatic heterocyclic compound to maintain the final product in a liquid phase until the reaction is completed.

For example, the basic metal oxide maintains a reactant including the aromatic heterocyclic compound raw material and the polar solvent at basic pH of 6 to 8, so the dicarboxylic acid aromatic heterocyclic compound may be maintained in a form of salt and thus in a liquid state, unless treated with acid.

Reaction Scheme 2 below is a state structural formula representing metal salts of FDCA, wherein a metal of the basic metal oxide, M₁ (when the metal is divalent) or M₂ (when the metal is trivalent), coordinates with FDCA, an example of the dicarboxylic acid aromatic heterocyclic compound, and thus keeps the FDCA in a liquid phase.

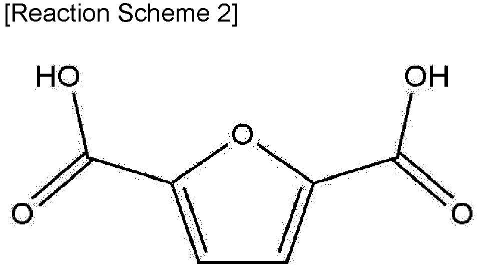

In addition, when the active metal is supported on a carbon carrier in the prior art, there may be an oxidation loss during the oxidation reaction, resulting in a carrier loss. Specifically, the carbon carrier may contribute to production of H₂O₂ during the oxidation reaction or be converted into CO or CO₂. On the contrary, when the basic metal oxide is used, the aforementioned side reactions may be reduced, improving a yield.

In addition, when the dicarboxylic acid aromatic compound is prepared, the reaction products may somewhat include 5-formyl-2-furoic acid (FFCA). In order to remove the FFCA, the reaction products are dissolved in water to dissolve and remove the FFCA in the water by breaking a double bond of a furan ring through a hydrogenation reaction of the FFCA but to purify FDCA not dissolved in the water. In addition, in order to filter Pd/C leaked in a batch reaction, the reaction products need to be dissolved in water, wherein the basic metal oxide may play a role of dissolving the dicarboxylic acid aromatic compound in the water.

The basic metal oxide capable of forming a complex by coordinate bonding with the dicarboxylic acid aromatic heterocyclic compound includes BaO, Nd₂O₃, Cs₂O, CsX (X is OH, Cl, Br, or I), basic zeolite, basic composite metal oxide, metal-organic frameworks (MOFs), hydrotalcite, hydroxyapatite, or a combination thereof.

On the other hand, the Mg oxide is difficult to mold into a spherical type or a granule type, but if molded, there is a problem of being easily broken. Accordingly, the Mg oxide is difficult to utilize as a catalyst carrier in the continuous process.

For example, the basic zeolite may be zeolite in which the hydrogen of a high silica beta or mordenite acidic zeolite having a silica/alumina (SiO₂/Al₂O₃) molar ratio of 20 to 1000 is substituted with an ion including Na, K, Rb, Cs, Mg, Ca, Sr, Ba, or a combination thereof.

For example, the basic composite metal oxide may include Mg₂Al₂O₄, ZnAl₂O₄, BaTiO₃, ZnTiO₃, MgO-SiO₂, CaO-SiO₂, SrO-SiO₂, BaO-SiO₂, MgO-Al₂O₃, CaO-Al₂O₃, SrO-Al₂O₃, BaO-Al₂O₃, MgO-TiO₂, CaO-TiO₂, SrO-TiO₂, BaO-TiO₂, MgO-ZnO, CaO-ZnO, SrO-ZnO, BaO-ZnO, or a combination thereof. The basic metals such as Na, Mg, or Ca, and the like react with some carriers at a high temperature, partially having a spinel structure. Accordingly, the catalyst have excellent durability and high activity against coke formation, thereby increasing reactivity and stability. In general, alumina has property of Lewis acid, and accordingly, this acidic property may increase the coke formation during the reaction. However, the catalyst may change the acidic property of the carrier to basic property to have the spinel structure, suppressing the coke formation.

For example, the metal-organic frameworks may include Zr-based MOFs, Mg-based MOFs, Ca-based MOFs, Sr-based MOFs, Ba-based MOFs, or a combination thereof.

For example, the hydrotalcite may include [Mg₄Al₂(OH)₁₂(CO₃)·4H₂O], [Mg₆Al₂(OH)₁₆(CO₃)·4H₂O], [Mg₄Zn₂Al₂(OH)₁₆(CO₃)·4H₂O], [Ca₄Al₂(OH)₁₂(NO₃)₂·xH₂O], [Ca₆Al₂(OH)₁₆(NO₃)₂·xH₂O], [Ca₄Zn₂Al₂(OH)₁₆(CO₃)·4H₂O], or a combination thereof.

An amount of the heterogeneous catalyst is not particularly limited but may be, for example, less than or equal to 600 moles or 50 moles to 600 moles with reference to an active metal in the catalyst based on 1 mole of the aromatic heterocyclic compound raw material represented by Chemical Formula 1.

The heterogeneous catalyst may be prepared by supporting the active metal on the basic metal oxide carrier. For example, a method of supporting the active metal on the basic metal oxide carrier may including impregnating the basic metal oxide carrier in a solution including an active metal precursor and then, optionally firing it.

Specifically, the active metal precursor is dissolved in a solvent to prepare a solution including the active metal precursor, and the basic metal oxide carrier is impregnated therein. The solvent may include water, alcohol, or a combination thereof.

When the active metal is ruthenium, the active metal precursor may be, for example, at least one selected from dimethyl ruthenium, diethyl ruthenium, ruthenium acetate, ruthenium acetate dihydrate, ruthenium acetylacetonate, ruthenium acetylacetonate hydrate, ruthenium iodide, ruthenium bromide, ruthenium chloride, ruthenium fluoride, ruthenium fluoride tetrahydrate, ruthenium carbonate, ruthenium cyanide, ruthenium nitrate, ruthenium nitrate hexahydrate, ruthenium oxide, ruthenium peroxide, ruthenium perchlorate, ruthenium perchlorate hexahydrate, ruthenium sulfate, diphenyl ruthenium, ruthenium naphthalate, ruthenium oleate, and ruthenium stearate. In addition, the same can be applied to the case where the active metal is not ruthenium.

The impregnation may be performed at 40 °C to 80 °C under a normal pressure. Subsequently, the impregnated material may be optionally fired at 40 °C to 800 °C or 100 °C to 600 °C. Herein, the pressure may be maintained as it is.

A method of preparing a dicarboxylic acid aromatic heterocyclic compound according to another embodiment includes subjecting an aromatic heterocyclic compound raw material to an oxidation reaction in a polar solvent in the presence of the heterogeneous catalyst to prepare a dicarboxylic acid aromatic heterocyclic compound.

The aromatic heterocyclic compound raw material may be an aromatic heterocyclic compound represented by Chemical Formula 1, and may include, for example, compounds represented by Chemical Formula 2 to Chemical Formula 5, or a combination thereof. Since the raw materials are as described above, repetitive descriptions are omitted.

The oxidation reaction may be performed at 80 °C to 200 °C under a normal pressure for 2 hours to 10 hours.

The polar solvent may be a known polar solvent, for example, dimethylsulfoxide, ethanol, acetonitrile, or the like. The polar solvent may be used in an amount of 10 parts by weight to 100 parts by weight based on 100 parts by weight of the aromatic heterocyclic compound raw material, but the present invention is not limited thereto.

A reaction product obtained through the step of preparing the dicarboxylic acid aromatic heterocyclic compound may be a crude composition including the aromatic heterocyclic compound raw material and the dicarboxylic acid aromatic heterocyclic compound.

For example, a target compound, which is the dicarboxylic acid aromatic heterocyclic compound, may be 2,5-furandicarboxylic acid (FDCA) represented by Chemical Formula 6.

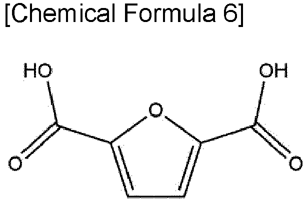

Herein, the reaction product may inevitably include the aromatic heterocyclic compound raw material represented by Chemical Formula 1 (for example, the compounds represented by Chemical Formula 2 to Chemical Formula 5).

In other words, the reaction is an oxidation reaction using the aforementioned raw materials under the heterogeneous catalyst, and an oxide of an aromatic heterocyclic compound obtained from the reaction (hereinafter, referred to as dicarboxylic acid aromatic heterocyclic compounds or reaction products) may mainly include 2,5-furandicarboxylic acid (FDCA), 2-carboxyl-5-formyl furan (FFCA), levulinic acid, or the like due to the oxidation of an unsaturated group included in the backbone, as shown in Reaction Scheme 1.

This product in general has a higher melting point than the aromatic heterocyclic compound raw material and thus are difficult to take out from the reactor in a molten state, requiring a post process such as purification, crystallization, or the like. For example, the 2-carboxyl-5-formyl furan may be purified by dissolving the reaction product in water to dissolve and remove the FFCA in the water by breaking a double bond of a furan ring through a hydrogenation reaction but to obtain FDCA not dissolved in the water.

Subsequently, the reaction product is titrated with acid such as HCl and the like, washed with water (DIW), and recrystallized, obtaining a purified dicarboxylic acid aromatic heterocyclic compound.

Hereinafter, specific examples of the invention are presented. However, the examples described below are only intended to specifically illustrate or explain the invention, and the scope of the invention should not be limited thereto.

### [Preparation Example: Preparation of Heterogeneous Catalyst]

### (Preparation Example 1: Preparation of 5 % Ru/BaO)

A Ru precursor (RuCl₃·3H₂O) as active metal component and BaO were stirred for 12 hours under an N₂ atmosphere, and NaNH₄ was dropped thereto 10 times as much as the Ru precursor and then, reacted under the N₂ atmosphere at room temperature for 24 hours, while stirred at 500 rpm, obtaining a 5% Ru/BaO catalyst.

### (Preparation Example 2: Preparation of 5% Ru/Nd₂O₃)

A Ru precursor (RuCl₃·3H₂O) as active metal component and Nd₂O₃ were stirred for 12 hours under an N₂ atmosphere, and NaNH₄ was dropped thereto 10 times as much as the Ru precursor and then, reacted under the N₂ atmosphere at room temperature for 24 hours, while stirred at 500 rpm, obtaining a 5% Ru/Nd₂O₃ catalyst.

### (Preparation Example 3: Preparation of 5 % Ru/basic zeolite)

A Ru precursor (RuCl₃·3H₂O) and basic zeolite (zeolite in which hydrogen of high silica beta acidic zeolite was substituted with Mg ions) as active metal components were stirred for 12 hours under an N₂ atmosphere, and NaNH₄ was dropped thereto 10 times as much as the Ru precursor and then, reacted under the N₂ atmosphere at room temperature for 24 hours, while stirred at 500 rpm, obtaining a 5% Ru/basic zeolite catalyst.

### (Preparation Example 4: Preparation of 5% Ru/Basic Zeolite Including a Bi Shell)

A Ru precursor (RuCl₃·3H₂O) as an active metal component, a Bi precursor (Bi(NO₃)₃·5H₂O) as a catalyst enhancer, and basic zeolite (zeolite in which hydrogen of high silica beta acidic zeolite was substituted with Mg ions) were stirred for 12 hours under an N₂ atmosphere, and NaNH₄ was dropped thereto 10 times as much as the Ru precursor and then, reacted under the N₂ atmosphere at room temperature for 24 hours, while stirred at 500 rpm, obtaining a 5% Ru/basic zeolite catalyst.

Magnesium nitrate 6hydrate (Mg(NO₃)₂·6H₂O) (Junsei Chemical Co., Ltd.) was added thereto and then, stirred for 1 hour at 200 rpm. Subsequently, the stirred Bi(NO₃)₃·5H₂O solution was treated with ammonia water to have pH 11, and the co-precipitated mixture was stirred for 1 hour at 200 rpm, preparing a uniform mixed solution. Then, the prepared mixed solution was put into a hydrothermal synthesizer for a hydrothermal process and synthesized at 200 °C for about 15 hours at 300 rpm, obtaining precipitates.

### (Comparative Preparation Example 1: Preparation of 5% Ru/C)

A 5% Ru/C catalyst was obtained in the same manner as in Example 1 except that the Ru precursor (RuCl₃·3H₂O) as active metal component and water-dispersed carbon were reacted under the same conditions as in Example 1.

### [Example: Oxidation Reaction Experiment]

### (Examples 1 to 4)

1.5 kg of each heterogeneous catalyst of Preparation Examples 1 to 4 was filled in a column having a diameter of 20 cm and a height of 100 cm made of a SUS material as a reactor. After the filling, the reactor was maintained at 150 °C under vacuum to remove adsorbed impurities.

As a raw material, 5-hydroxymethyl furfural (HMF) represented by Chemical Formula 2 was prepared through conversion from sugar and dissolved at a concentration of 1 wt% in acetonitrile as a polar solvent, and 5 L of the solution was filled in a raw material storage tank. After the filling, the solution was pressurized to 3 bar with oxygen gas.

The raw material was transported to the column to contact the catalyst in the column, obtaining a crude composition including an oxide.

### (Comparative Example 1: Oxidation Reaction Experiment)

An oxidation reaction was experimented in the same manner as in Example 1 except that the heterogeneous catalyst of Comparative Preparation Example 1 was used as the heterogeneous catalyst.

### [Experimental Example: Oxidation Reaction Experiment]

The crude compositions of Examples 1 to 4 and Comparative Example 1 were measured with respect to a yield (%) of FDCA at each reaction time through component analysis, and the results are shown in Table 1 below. Table 1 shows FDCA yields (%) according to types of catalysts.

**(Table 1)**

| | Type of catalyst | Reaction time (h) | | | |
|---|---|---|---|---|---|
| | | 3 | 4 | 5 | 6 |
| Example 1 | 5% Ru/BaO | 45.00% | 50.00% | 54.50% | 60.50% |
| Example 2 | 5% Ru/Nd₂O₃ | 35.50% | 40.00% | 45.00% | 55.00% |
| Example 3 | 5% Ru/basic zeolite | 65.00% | 69.00% | 72.00% | 73.00% |
| Example 4 | 5% Ru/basic zeolite including Bi shell | 90.00% | 93.00% | 95.00% | 95.00% |
| Comparative Example 1 | 5% Ru/C | 0.14% | 0.56% | 0.90% | 1.50% |

Referring to Table 1, when Ru/C was used for the FDCA synthesis, a reaction yield was significantly low. The reason is that FDCA crystallized in a catalyst layer inside the continuous reactor was extracted and attached to the Ru/C catalyst. As a result, since an internal pressure of the reactor was significantly increased, FDCA in powder form did not escape out of the reactor, significantly resulting in the low yield.

However, when a basic metal oxide carrier was used, since the carrier coordinated with FDCA and thus maintained the reactant in a basic state of pH 6 or higher, FDCA was not crystallized but obtained in a liquid phase. The FDCA liquid was adjusted to have pH 3 or less through acid titration to obtain crystallized powder.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

### [Industrial Applicability]

The present invention relates to a catalyst for preparing a dicarboxylic acid aromatic heterocyclic compound and a method for preparing the dicarboxylic acid aromatic heterocyclic compound, wherein the catalyst may be used, for example, in a reaction that HMF is oxidized to prepare FDCA.

## Claims

1. A heterogeneous catalyst for preparing a dicarboxylic acid aromatic heterocyclic compound, comprising
an active metal, and
a basic metal oxide carrier capable of carrying the active metal and forming a complex by coordinate bonding with the dicarboxylic acid aromatic heterocyclic compound.

2. The heterogeneous catalyst of claim 1, wherein
the active metal includes Fe, Ni, Ru, Rh, Pd, Os, Ir, Au, Pt, or a combination thereof.

3. The heterogeneous catalyst of claim 1, wherein
the heterogeneous catalyst includes 0.1 wt% to 10 wt% of the active metal based on the total weight of the heterogeneous catalyst.

4. The heterogeneous catalyst of claim 1, wherein
the heterogeneous catalyst includes a catalyst enhancer including Bi, Na, Cu, Ce, K, or a combination thereof.

5. The heterogeneous catalyst of claim 4, wherein
the heterogeneous catalyst includes a shell including the catalyst enhancer on the surface of the active metal.

6. The heterogeneous catalyst of claim 1, wherein
the basic metal oxide capable of forming a complex by coordinate bonding with the dicarboxylic acid aromatic heterocyclic compound includes BaO, Nd₂O₃, Cs₂O, CsX (X is OH, Cl, Br, or I), basic zeolite, basic composite metal oxide, metal-organic frameworks (MOFs), hydrotalcite, hydroxyapatite, or a combination thereof.

7. The heterogeneous catalyst of claim 6, wherein
the basic zeolite is a zeolite in which hydrogen of a high silica beta or mordenite acidic zeolite having a silica/alumina (SiO₂/Al₂O₃) molar ratio of 20 to 1000 is substituted with an ion including Na, K, Rb, Cs, Mg, Ca, Sr, Ba, or a combination thereof.

8. The heterogeneous catalyst of claim 6, wherein
the basic composite metal oxide includes Mg₂Al₂O₄, ZnAl₂O₄, BaTiO₃, ZnTiO₃, MgO-SiO₂, CaO-SiO₂, SrO-SiO₂, BaO-SiO₂, MgO-Al₂O₃, CaO-Al₂O₃, SrO-Al₂O₃, BaO-Al₂O₃, MgO-TiO₂, CaO-TiO₂, SrO-TiO₂, BaO-TiO₂, MgO-ZnO, CaO-ZnO, SrO-ZnO, BaO-ZnO, or a combination thereof.

9. The heterogeneous catalyst of claim 6, wherein
the metal-organic frameworks include Zr-based MOFs, Mg-based MOFs, Ca-based MOFs, Sr-based MOFs, Ba-based MOFs, or a combination thereof.

10. The heterogeneous catalyst of claim 6, wherein
the hydrotalcite includes [Mg₄Al₂(OH)₁₂(CO₃)·4H₂O], [Mg₆Al₂(OH)₁₆(CO₃)·4H₂O], [Mg₄Zn₂Al₂(OH)₁₆(CO₃)·4H₂O], [Ca₄Al₂(OH)₁₂(NO₃)₂·xH₂O], [Ca₆Al₂(OH)₁₆(NO₃)₂·xH₂O], [Ca₄Zn₂Al₂(OH)₁₆(CO₃)·4H₂O], or a combination thereof.

11. The heterogeneous catalyst of claim 1, wherein
the heterogeneous catalyst produces a dicarboxylic acid aromatic heterocyclic compound represented by Chemical Formula 6 by oxidizing an aromatic heterocyclic compound represented by Chemical Formula 2 in a polar solvent:

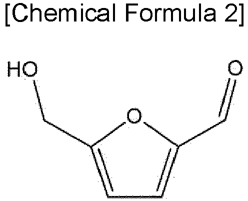

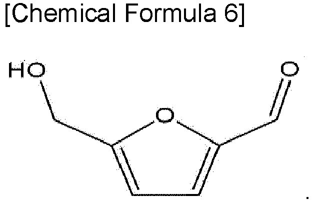

12. A method of preparing a dicarboxylic acid aromatic heterocyclic compound, comprising
preparing a dicarboxylic acid aromatic heterocyclic compound by oxidizing a raw material for an aromatic heterocyclic compound in a polar solvent in the presence of a heterogeneous catalyst,
wherein the heterogeneous catalyst includes an active metal, and a basic metal oxide carrier capable of carrying the active metal and forming a complex by coordinate bonding with the dicarboxylic acid aromatic heterocyclic compound.

13. The method of preparing the dicarboxylic acid aromatic heterocyclic compound of claim 12, wherein
the aromatic heterocyclic compound raw material is represented by Chemical Formula 1: 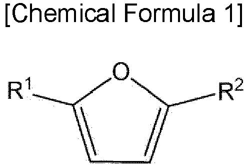
wherein, in Chemical Formula 1, one of R¹ and R² is an aldehyde group, and the other is a hydrogen group, a hydroxyl group, an aldehyde group, an acetoxy group, a halogen group, a substituted or unsubstituted C₁-C₂₀ alkyl group, or a substituted or unsubstituted C₁-C₂₀ alkoxy group.

14. The method of preparing the dicarboxylic acid aromatic heterocyclic compound of 13, wherein
the aromatic heterocyclic compound raw material represented by Chemical Formula 1 includes a compound represented by Chemical Formula 2, a compound represented by Chemical Formula 3, a compound represented by Chemical Formula 4, a compound represented by Chemical Formula 5, or a combination thereof.

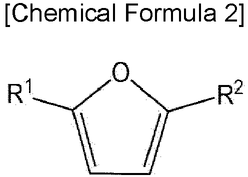

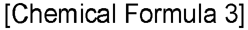

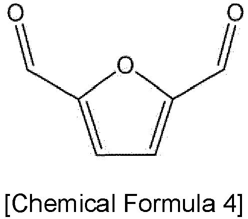

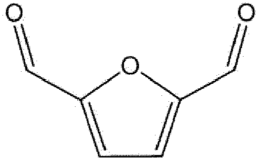

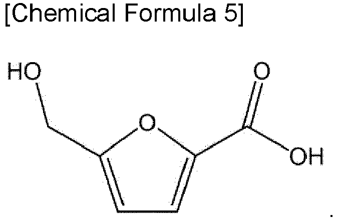

15. The method of preparing the dicarboxylic acid aromatic heterocyclic compound of claim 12, wherein
the dicarboxylic acid aromatic heterocyclic compound is 2,5-furandicarboxylic acid (FDCA) represented by Chemical Formula 6:
